(19) **Europäisches Patentamt**
**European Patent Office**
**Office européen des brevets**

(11) **EP 1 431 264 B1**

(12) **EUROPÄISCHE PATENTSCHRIFT**

(45) Veröffentlichungstag und Bekanntmachung des Hinweises auf die Patenterteilung:
**20.01.2010 Patentblatt 2010/03**

(51) Int Cl.:
*C07C 29/04* (2006.01)   *C07C 31/12* (2006.01)

(21) Anmeldenummer: **03103901.9**

(22) Anmeldetag: **22.10.2003**

(54) **Verfahren zur Herstellung von tert.-Butanol**

Process for the preparation of tert.-butanol

Procédé pour la préparation de tert.-butanol

(84) Benannte Vertragsstaaten:
**AT BE BG CH CY CZ DE DK EE ES FI FR GB GR HU IE IT LI LU MC NL PT RO SE SI SK TR**

(30) Priorität: **19.12.2002 DE 10259413**
**08.07.2003 DE 10330710**

(43) Veröffentlichungstag der Anmeldung:
**23.06.2004 Patentblatt 2004/26**

(73) Patentinhaber: **Evonik Oxeno GmbH**
**45772 Marl (DE)**

(72) Erfinder:
 • **Scholz, Bernhard**
 **45768, Marl (DE)**
 • **Nierlich, Franz**
 **45768, Marl (DE)**
 • **Reusch, Dieter**
 **45772, Marl (DE)**
 • **Santiago-Fernandez, Silvia**
 **46049, Oberhausen (DE)**
 • **Beckmann, Andreas**
 **45657, Recklinghausen (DE)**
 • **Büschken, Wilfried**
 **45721, Haltern am See (DE)**
 • **Kaizik, Alfred**
 **45772, Marl (DE)**

(74) Vertreter: **Hirsch, Hans-Ludwig et al**
**Evonik Degussa GmbH**
**DG-IPM-PAT - Bau 1042 / PB 15**
**Paul-Baumann-Strasse 1**
**45764 Marl (DE)**

(56) Entgegenhaltungen:
**US-A- 4 307 257    US-A- 6 111 148**

**EP 1 431 264 B1**

## Beschreibung

Die Erfindung betrifft ein Verfahren zur Herstellung von tert.-Butanol durch Anlagerung von Wasser an Isobuten in Gegenwart eines saueren Ionenaustauschers.

Tert.-Butanol (TBA) ist ein wichtiges großtechnisch hergestelltes Produkt und wird als Lösungsmittel und als Zwischenprodukt für die Herstellung von Methacrylsäuremethylester verwendet. Es ist Vorstufe für die Herstellung von Peroxiden, wie Peroxiketale, Perester oder Dialkylperoxide, mit mindestens einer tertiären Butylgruppe. Diese Verbindungen werden als Oxidationsmittel und als Starter für Radikalreaktionen, wie beispielsweise Olefinpolymerisation oder Vernetzung von Kunststoffen, eingesetzt. Als Zwischenstufe dient tert.-Butanol zur Gewinnung von reinem Isobuten aus Isobutengemischen. Darüber hinaus ist es ein Reagenz zur Einführung von tertiären Butylgruppen. Seine Alkalisalze sind starke Basen, die in vielen Synthesen Verwendung finden.

Tertiäres Butanol kann durch sauer katalysierte Anlagerung von Wasser an Isobuten erhalten werden. Technische Isobutengemische enthalten häufig auch andere Olefine, wie 2-Butene. Werden diese Edukte eingesetzt, so wenden technische Verfahren Bedingungen an, bei denen fast ausschließlich das Isobuten, nicht aber die anderen Olefine hydratisiert werden und Nebenreaktionen, wie Homo- oder Heterooligomerisierung der Olefine, nahezu vollständig unterdrückt werden. Solche Prozesse arbeiten gewöhnlich in der flüssigen Phase und können in zwei Gruppen unterteilt werden: a) Verfahren, bei denen die Umsetzung in einer wässrigen Katalysatorlösung erfolgt und b) heterogene katalytische Prozesse, bei denen feste, in der Reaktionsphase unlösliche Katalysatoren eingesetzt werden.

Homogene katalytische Verfahren verwenden als Katalysator Schwefelsäure, Heteropolysäuren, p-Toluolsulfonsäure, oder andere starke Säuren. Diese homogenen Katalysatoren mit hoher Aktivität bilden mit dem Reaktionsprodukt gewöhnlich eine homogene Phase, so dass der Katalysator nicht mechanisch abgetrennt werden kann. Bei manchen Verfahren wird zusätzlich ein Lösungsmittel verwendet. Wird das tertiäre Butanol durch Destillation aus der Reaktionslösung gewonnen, wird die Ausbeute durch die Rückreaktion und die Bildung von Nebenprodukten gemindert. Für heterogene katalysierte Verfahren werden häufig saure Ionenaustauscher als Katalysatoren eingesetzt.

In EP 0 579 153 wird ein Verfahren beschrieben, bei dem eine wässrige Lösung eines sulfonierten Polymer (Polystyrolsulfonsäuren oder Polyvinylsulfonsäuren mit Molmassen von 1000 bis 100 000 g/mol ) als Katalysator verwendet wird. Isobuten bzw. das Isobuten-haltige Edukt und die Katalysatorphase durchströmen den Reaktor im Gegenstrom. Der Reaktionsaustrag ist zweiphasig und trennt sich in eine tert.-Butanol enthaltene organische Oberphase und die Katalysatorphase. Die organische Phase wird destillativ zu tert.-Butanol aufgearbeitet. Die Katalysatorphase wird nach Ergänzung des verbrauchten Wassers in den Reaktor zurückgeführt.

Die Hydratisierung von Isobuten zu tert.-Butanol mit Hilfe fester, weder in den Edukten noch in den Produkten löslicher, saurer Katalysatoren hat den Vorteil, dass das Reaktionsgemisch säurefrei ist und ohne Verluste durch Rückspaltung oder durch andere Nebenreaktionen zu tert.-Butanol aufgearbeitet werden kann. Die Reaktion läuft an der Oberfläche des Katalysators ab. Damit eine Reaktion zustande kommt, müssen beide Reaktionspartner zur gleichen Zeit an der aktiven Stelle des Katalysators sein. Dies wird dadurch erschwert, dass Wasser und Isobuten bzw. ein Isobuten-haltiges Kohlenwasserstoffgemisch nicht miteinander mischbar sind. Um akzeptable Umsätze zu erhalten, werden hier Solventien verwendet, die ein homogenes Gemisch aus Wasser und Isobuten-Einsatzgemisch ermöglichen.

In DE 30 31 702 wird Methanol für diesen Zweck als Lösungsmittel sowohl für Wasser als auch für Isobuten bzw. für ein Isobuten-haltiges Kohlenwasserstoffgemisch beschrieben. Als Produkte werden tert.-Butanol und Methyltert.-butylether nebeneinander erhalten.

In EP 0 010 993 werden aliphatische Carbonsäuren mit 1 bis 6 C-Atomen als Lösungsmittel für beide Edukte eingesetzt. Dabei entstehen als Nebenprodukte die tertiären Butylester dieser Säuren. Diese müssen zu tert.-Butanol und Carbonsäuren hydrolisiert werden.

In DE 30 31 702 werden Sulfolane und in US 4 327 231 mehrwertige Alkohole des Neo-Typs, wie beispielsweise Neopentylglykol, eingesetzt. Diese Lösungsmittel müssen vom tert.-Butanol abgetrennt werden. Darüber hinaus besteht die Gefahr, dass das verwendete Lösungsmittel im Dauerbetrieb einer solchen Anlage zersetzt wird.

In WO 99/33775 wird ein Verfahren zur Herstellung von tert.-Butanol durch Umsetzung einer Mischung, bestehend aus Wasser, tert.-Butanol und Isobuten bzw. einem Isobuten-haltigen Kohlenwasserstoffbemisch, an einem Kationenaustauscherharz in einem Reihen-Multistufen-Reaktor beschrieben. Die Reaktionstemperatur in den einzelnen Reaktoren liegt unter 65 °C. Ein Teil des Zwischenprodukts aus dem ersten Reaktor wird in den Eingang des gleichen Reaktors zurückgeführt. Die Zirkulationsrate (die Menge an Zwischenproduktgemisch, die wieder in den ersten Reaktor zurückgeleitet wird, als Verhältnis zum Einsatzgemisch) beträgt hierbei 1,8 bis 10 und der Gewichtsanteil an tert.-Butylalkohol gegenüber dem Kohlenwasserstoffgemisch (Summe aus Isobuten und gegebenenfalls anderen Kohlenwasserstoffen) am Eingang des ersten Reaktors 0,5 bis 3,5. Das nicht zurückgeführte Gemisch aus dem ersten Reaktor durchströmt ohne Zwischeneinspeisung von Wasser zwei weitere Reaktoren im geraden Durchgang. Das Rohprodukt aus dem letzten Reaktor wird destillativ aufgearbeitet. Gegebenenfalls wird ein Teil des gewonnenen tert.-Butanol in den ersten Reaktor zurückgeführt.

Nachteilig an diesem Verfahren ist die geringe Raum-Zeit-Ausbeute. Die Produktionsrate an tert.-Butanol liegt im ersten Reaktor (Betrieb unter Produktrückführung) nach den Beispielen 1 bis 6 zwischen 0,078 und 0,085 kg je Stunde je Liter Katalysator (bei einer Isobuten-Eingangskonzentration zwischen 8,3 und 16,2 Massen-% und Umsätzen zwischen 60,2 und 66,1%).

In DE 30 25 262 wird ein Verfahren zur Herstellung von TBA unter Einsatz eines Dreistoffgemischs Wasser/Isobuten/TBA beschrieben.

Das Verfahren wird bevorzugt an der Mischungslücke dieses Systems durchgeführt, kann aber auch in einem heterogenen und homogenen Bereich um die Mischungslücke ausgeübt werden.

Der Arbeitsbereich des Verfahrens ist durch ein Dreiecksdiagramm beschrieben, wobei auf Grund des ternären Gemisches sowohl bei heterogener wie homogener Phase eine große Variabilität der Zusammensetzung des Gemischs besteht.

Die Zusammensetzungen dieses Gemisches im homogenen, d. h. einphasigen Bereich, beginnen bei den Zusammensetzungen mit maximalem Wasseranteil (Löslichkeitsgrenze des Wassers im Gemisch, erreichbar durch Wasserabscheider) und enden bei Mischungen mit einem geringen Wasseranteil (kleiner als ca. 5 % bezogen auf die Löslichkeitsgrenze).

Das ternäre Gemisch Isobuten-haltiger Kohlenwasserstoff/Wasser/tert.-Butanol mit einem schwankenden Isobutenanteil in Kohlenwasserstoffgemisch lässt daher auch unter Zuhilfenahme von DE 30 25 262 eine große Anzahl von Kombinationsmöglichkeiten offen, um möglichst selektiv und mit ausreichenden Reaktionsgeschwindigkeiten unter heterogener Katalyse tert.-Butanol herzustellen. Um jedoch hohe Reaktionsgeschwindigkeiten und Selektivitäten zu erhalten, werden in DE 30 25 262 bevorzugt Gemische eingesetzt, die bezüglich ihrer Wassergehalte nahe an der Löslichkeitsgrenze liegen.

Da die bekannten Verfahren, die saure Kationenaustauscherharze als Katalysator einsetzen, zwar eine einfache Aufarbeitung bieten, jedoch nicht hinsichtlich der Raum-Zeit-Ausbeute und/oder Selektivität befriedigen, bestand die Aufgabe, ein Verfahren bereitzustellen, das es ermöglicht, tert.-Butanol aus Isobuten oder Isobuten-haltigen Gemischen mit guten Raum-Zeit-Ausbeuten und guter Selektivität bei möglichst hohem Isobuten-Umsatz zu erhalten.

Es wurde überraschend gefunden, das Reaktionsgeschwindigkeit, Selektivität und Raum-Zeit-Ausbeute bei der Umsetzung eines homogenen Gemisches, bestehend aus Wasser, tert.-Butanol und Isobuten bzw. einem Isobuten-haltigen Kohlenwasserstoff-Gemisch, an einem sauren Ionenaustauscherharz zu tert.-Butanol vergrößert werden kann, wenn das Einsatzgemisch weniger als die aufgrund der Löslichkeit des Wassers im ternären Gemisch mögliche Wassermenge enthält.

Gegenstand der vorliegenden Erfindung ist daher ein Verfahren zur Herstellung von tert.-Butanol durch Umsetzung eines homogenen Reaktionsgemisches aus Wasser, tert.-Butanol und einem Isobuten-haltigen Kohlenwasserstoffgemisch, an einem sauren Ionenaustauscherharz bei 30 bis 120 °C, wobei das homogene Reaktionsgemisch bei Beginn der Umsetzung einen Massenanteil von Isobuten von über 10 Massen-% und einen Wasseranteil von 30 bis 80 % der durch die Löslichkeit von Wasser im Reaktionsgemisch möglichen Wassermenge enthält.

Bevorzugt ist der Wasseranteil im Reaktionsgemisch geringer, wie z. B. 50 - 80 %, 60 - 30 % oder 70 - 80 %. Diese Angaben beziehen sich jeweils auf die Maximalmenge des durch die Löslichkeit von Wasser im homogenen Reaktionsgemisch definierten Hauptmenge.

Nach dem Massenwirkungsgesetz für die Reaktion von Isobuten mit Wasser zu TBA gilt Gleichung 1:

$$C_B = K * C_I * C_W \qquad\qquad (\text{Gl. 1})$$

$C_B$: molare Konzentration von tert.-Butanol im Reaktionsgemisch
$C_I$ : molare Konzentration von Isobuten im Reaktionsgemisch
$C_W$ : molare Konzentration von Wasser im Reaktionsgemisch
K : Gleichgewichtskonstante

Für die Reaktionsgeschwindigkeit der tert.- Butanol- Bildung bei der Umsetzung einer homogenen tert.-Butanol/Wasser/Isobuten-Lösung gilt folgende Gleichung (E. Velo, L. Puigjaner, F. Recasens, Inhibition by Product in the Liquid-Phase-Hydration of Isobutene to tert-Butyl Alcohol : Kinetic and Equilibrium Studies, Ind. Eng. Chem.Res. 1988, 27, 2224-2231):

$$r = k\,(\,C_I * C_W - C_B/K\,) : (\,1 + c * C_B)^n \qquad n = 1 \text{ oder } 2 \quad (\text{Gl. 2})$$

r : Reaktionsgeschwindigkeit

k und c sind Konstanten bei vorgegebener Temperatur, Katalysator und Katalysatormenge.

Gemäß dieser Gleichung steigt die Reaktionsgeschwindigkeit der tert-Butanolbildung mit zunehmender Wasserkonzentration an. Dementsprechend wird in WO 99/33775 und DE 030 25 262 bei Einsatz einer homogenen Lösung bevorzugt die maximal mögliche Wassermenge eingesetzt.

Umso überraschender ist der Befund, dass das Maximum der Reaktionsgeschwindigkeit der tert.-Butanolbildung bei gleichem Isobuten/tert.-Butanol-Verhältnis nicht bei Einsatz einer mit Wasser gesättigten Lösung, sondern bei Gemischen mit geringerem Wassergehalt erreicht wird.

Bei der Umsetzung eines homogenen Gemisches aus einem Isobuten-haltigen Kohlenwasserstoffgemisch (z. B. Raffinat I), tert.-Butanol und Wasser an einem sauren Ionenaustauscherharz ist bei gleichbleibenden Parametern die Reaktionsgeschwindigkeit für die tert.-Butanol-Bildung zeitabhängig. Abhängig vom Wassergehalt des eingesetzten Ionenaustauscherharzes kann die Anfangsgeschwindigkeit der TBA-Bildung fallen oder steigen. Ausgehend von einem Harz mit geringen Wassergehalt verringert sich die Anfangsgeschwindigkeit, die Geschwindigkeit für die tert.-Butanol-Bildung nimmt erst nach 2 bis 6 Tagen einen konstanten Wert an. In der gleichen Zeit nimmt der Katalysator Wasser auf, wie sich aus der Wasserbilanz leicht feststellen lässt. Die Wassermenge, die vom Katalysator aufgenommen wird, ist von der Wasserkonzentration im Reaktionsgemisch und von der Entfernung der Wasserkonzentration von der Löslichkeitsgrenze abhängig. Je näher die Wasserkonzentration an der Löslichkeitsgrenze liegt, desto mehr Wasser nimmt der Katalysator auf. Mit zunehmender Wasseraufnahme des Katalysators geht unter sonst gleichen Bedingungen die Reaktionsgeschwindigkeit der tert.-Butanol-Bildung zurück. Ursache dafür könnte die Herabsetzung der Säurestärke der aktiven Zentren durch Hydratisierung und/oder eine Hemmung des Stoffübergangs, insbesondere für Isobuten, durch die wasserhaltige Quellschicht des Katalysators sein. Der Wassergehalt im Einsatzgemisch beeinflusst die Reaktionsgeschwindigkeit für die tert.-Butanol-Bildung im Dauerbetrieb also in zweifacher Hinsicht, nämlich durch den Konzentrationseffekt (siehe Gleichung 2), der die Geschwindigkeit erhöht, und durch die Wasseraufnahme des Katalysators, der die Geschwindigkeit herabsetzt.

Für die Wirtschaftlichkeit eines technisch ausgeübten Verfahrens sind nicht die Anfangsgeschwindigkeiten, sondern die Geschwindigkeit im stationären Zustand, d. h. im Dauerbetrieb einer Anlage relevant.

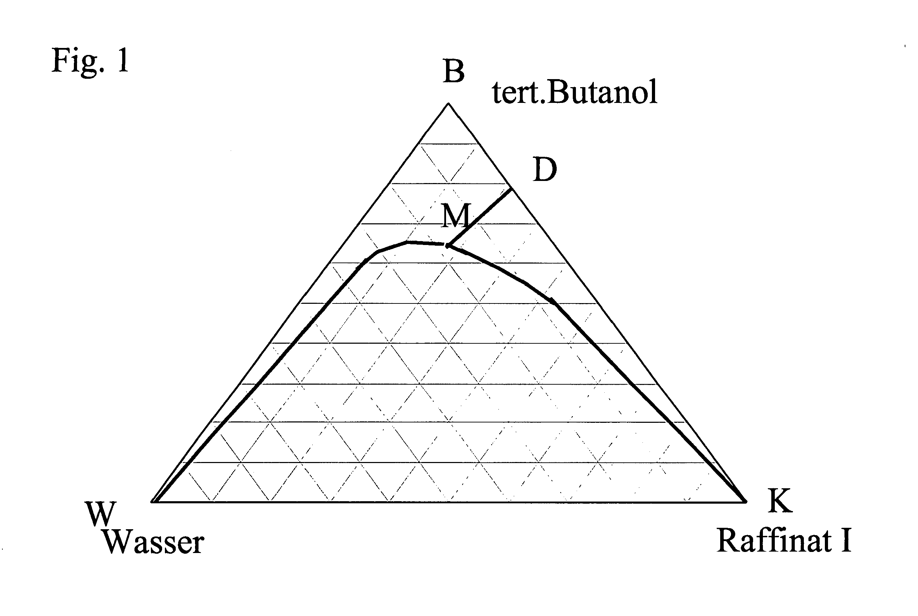

Die Zusammensetzung eines homogenen Gemisches für das erfindungsgemäße Verfahren wird mit Hilfe eines Phasendiagramms verdeutlicht. In Figur 1 ist das Phasendiagramm für Wasser, tert.-Butanol und einem Isobuten-haltigen Kohlenwasserstoffgemisch in einem Dreieckkoordinatensystem (Koordinaten 0 - 100 Massen-%) dargestellt. Das Kohlenwasserstoffgemisch (Raffinat I) besteht aus 45 % Isobuten und aus 55 % gesättigten und einfach ungesättigten $C_4$-Kohlenwasserstoffen. Das Phasendiagramm gilt für einen Temperaturbereich von 20 bis 80 °C und einem Druck, der über dem Sättigkeitsdruck des Kohlenwasserstoffgemisches liegt. In diesem Bereich besteht praktisch keine Druck- und Temperaturabhängigkeit. Bei anderer Zusammensetzung des Isobuten-haltigen Kohlenwasserstoffgemisches verändert sich dieses Diagramm nur wenig, sodass es sich für die Erläuterung der Erfindung eignet. Die Kurve von einer Dreiecksspitze W über den Scheitelpunkt M zu anderen Dreiecksspitze K stellt die Grenzlinie zwischen dem homogenen und heterogenen Bereich dar. Unterhalb der Kurve ist das Gemisch heterogen, oberhalb der Kurve ist der Bereich der homogenen Lösungen. Die Kurve gibt auch den maximalen Anteil an Wasser im Dreikomponentensystem bei Vorliegen einer homogenen Lösung wieder.

Im erfindungsgemäßen Verfahren hat der Reaktorzulauf bevorzugt eine Zusammensetzung, die in Figur 1 in der Fläche liegt, die durch den Kurvenabschnitt KM, durch die Strecke MD und durch die Strecke DK definiert ist. Die exakte Zusammensetzung des Gemisches bei Beginn der Reaktion liegt auf Grund des erfindungsgemäß geringen Wasseranteils noch weiter innerhalb dieser Fläche. MD ist das Lot von M auf die Basislinie KB. Sie gibt die Gemischzusammensetzung wieder, wenn ausgehend von Punkt M bei gleichbleibenden Kohlenwasserstoff/tert.-Butanol-Verhältnis die Wasserkonzentration verringert wird.

Im Temperaturbereich 40 bis 90 °C kann der maximale Wassergehalt (Löslichkeitskurve) für das Dreikomponentengemisch (tert.-Butanol/ Wasser/ Raffinat I mit 45 % Isobuten) nach den unten stehenden empirischen Gleichungen für den interessierenden Bereich (Linie KM) berechnet werden. Diese Gleichungen gelten auch, wenn das Isobuten-haltige Kohlenwasserstoffgemisch eine andere Zusammensetzung aufweist

$$\ln X_W = 0{,}0570\, X_B - 0{,}8215 \qquad\qquad (\text{Gl. 3a})$$

oder

$$X_W = \mathrm{EXP}\,[0,0570\,X_B - 0,8215] \qquad\qquad (\,\mathrm{Gl.\ 3b})$$

mit

$X_W$: Wassergehalt in Massen-%
$X_B$ : tert.-Butanolgehalt in Massen-%

Im erfindungsgemäßen Verfahren ist dementsprechend der Wassergehalt in der homogenen Reaktorzuflusslösung geringer als maximal möglich wäre ( Strecke KM). Er beträgt 30 bis 80 %, insbesondere 50 bis 80 %, 60 - 80 % oder 70 - 80 % der durch die Löslichkeit von Wasser im Gemisch von tert.-Butanol und dem Isobuten-haltigen Kohlenwasserstoffgemisch möglichen Wassermenge und kann durch einfache Versuche ermittelt oder nach Gleichung 3 a/b berechnet werden.

Erfindungsgemäß beträgt die Isobutenkonzentration im Reaktorzufluss über 10 Massen-%, insbesondere über 15 Massen-%. Die Reaktorzuflüsse sind also Gemische, deren Zusammensetzungen im bevorzugten Temperaturbereich von 35 °C bis 70 °C weit vom thermodynamischen Gleichgewicht zwischen Isobuten, Wasser und TBA entfernt sind.

Als Ausgangsstoff kann ein Isobuten-haltiges Kohlenwasserstoffgemisch, aber auch reines Isobuten eingesetzt werden. Bevorzugt enthält das Isobuten-haltige Kohlenwasserstoffgemisch keine Acetylenderivate, weniger als 5000 ppm an Dienen und keine weiteren Olefine mit einer oder mehreren Verzweigung(en) an der olefinischen Doppelbindung.

Technische Gemische, die Isobuten enthalten, sind beispielsweise Leichtbenzinfraktionen aus Raffinerien, $C_4$-Fraktionen aus FCC- oder Steamcracker, Gemische aus Fischer-Tropsch-Synthesen, Gemische aus Dehydrierung von Butanen, Gemische aus Skelettisomerisierung linearer Butene, Gemische, entstanden durch Metathese von Olefinen oder anderen technischen Prozessen.

Diese Gemische werden ggf. nach Entfernung der mehrfach ungesättigten Verbindungen im erfindungsgemäßen Verfahren eingesetzt. Beispielsweise kann die Gewinnung eines geeigneten Isobutengemisches aus der $C_4$-Fraktion eines Steamcrackers durch Extraktion des Butadiens oder durch dessen Selektivhydrierung zu linearen Butenen erfolgen. Dieser Einsatzstoff (Raffinat I bzw. selektivhydriertes Crack-$C_4$) besteht aus n-Butan, Isobutan, den drei linearen Butenen und Isobuten und ist ein bevorzugtes Edukt für das erfindungsgemäße Verfahren.

Optional könnte Raffinat I, hydriertes Crack-$C_4$ oder ein ähnlich zusammengesetztes Kohlenwasserstoffgemisch in einer Reaktivkolonne hydroisomerisiert werden. Dabei kann ein Gemisch aus Isobuten, (ggf. 1-Buten) und Isobutan gewonnen werden.

Die Konzentration des Isobutens im Kohlenwasserstoffgemisch kann in weitem Bereich variieren. Es ist jedoch wegen der Wirtschaftlichkeit des Verfahrens ratsam, Kohlenwasserstoffgemische mit einer Isobutenkonzentration größer 30 Massen-%, vorzugsweise größer 40 Massen-% einzusetzen.

Als Katalysator wird ein saurer Ionenaustauscher, der weder im Einsatzstoffgemisch noch im Produktgemisch löslich ist, eingesetzt. Der Katalysator darf unter Reaktionsbedingungen weder durch Hydrolyse noch durch andere Reaktionen saure Stoffe an das Produktgemisch abgeben, weil dies zu Ausbeuteverlusten bei der Aufarbeitung des Reaktionsgemisch führen würde.

Für die Aktivität der geeigneten Katalysatoren gilt, dass sie unter Reaktionsbedindungen die Hydratisierung von Isobuten bewirken, jedoch kaum die von nicht verzweigten Olefinen. Weiterhin dürfen sie die Oligomerisierung von Olefinen kaum katalysieren.

Eine geeignete Gruppe von Katalysatoren sind feste Ionenaustauscherharze mit Sulfonsäuregruppen. Besonders geeignete Ionenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung von Ionenaustauscherharzen mit Sulfongruppen verwendet. Die Harze können gelförmig, makroporös oder schwammförmig hergestellt werden. Geeignete Harze des Styrol-Divinylbenzol-Typs werden u. a. unter folgenden Handelsnamen verkauft: Duolite C20, Duolite C26, Amberlyst 15, Amberlyst 35, Amberlite IR-120, Amberlite 200, Dowex 50, Lewatit SPC 118, Lewatit SPC 108, Lewatit K2611 (Fa. Bayer), Lewatit K2631, OC 1501 (Fa. Bayer), Lewatit K2621, Lewatit K2629, Lewatit K2431.

Die Eigenschaften dieser Harze, insbesondere spezifische Oberfläche, Porosität, Stabilität, Quellung bzw. Schrumpfung und Austauschkapazität, können durch den Herstellprozess variiert werden.

Im erfindungsgemäßen Verfahren werden die Ionenaustauscherharze bevorzugt in ihrer H-Form verwendet. Die Ionenaustauscherkapazität liegt zwischen 2 bis 7, insbesondere 3 bis 6 eq/kg (bezogen auf feuchtes handelsübliches Harz).

Es werden vorzugsweise makroporöse Harze eingesetzt, wie beispielsweise Lewatit SPC 118 , Lewatit SPC 108 oder Lewatit K2631.

Die Korngröße des technischen Harzes liegt im Allgemeinen zwischen 0,5 und 2 mm. Die Korngrößenverteilung kann enger oder weiter gewählt werden. So können beispielsweise Ionenaustauscherharze mit sehr einheitlicher Korngröße (monodisperse Harze) eingesetzt werden.

Bei der Verwendung mehrerer Reaktoren können diese mit Harz gleicher oder unterschiedlicher Korngröße (bzw. Korngrößenverteilung) gefüllt sein.

Optional können die Ionenaustauscherharze als Formkörper, wie beispielsweise Zylinder, Ringe oder Kugeln, eingesetzt werden.

Es kann vorteilhaft sein, in Reaktoren, die mit hohen Lineargeschwindigkeiten durchströmt werden, zur Verringerung des Differenzdruckes ein größeres Korn einzusetzen, und in Reaktoren, die mit einer geringen Lineargeschwindigkeit durchströmt werden, zur Erzielung des optimalen Umsatzes ein kleineres Korn einzusetzen.

Um zu verhindern, dass das Harz während des Betriebs saure Gruppen abspaltet und somit Störungen im Aufarbeitungsteil des Verfahren verursachen könnte und um eine hohe Aktivität des Katalysators über einen langen Zeitraum beizubehalten, kann das Ionenaustauscherharz vorbehandelt werden, beispielsweise durch Spülen mit Wasser, TBA oder TBA/Wasser-Gemischen im Temperaturbereich von 40 bis 120 °C.

Da durch die Hydratisierung von Isobuten Wasser verbraucht wird, sinkt der Wassergehalt in der Reaktionsgemischung. Um eine möglichst hohe Ausbeute und Reaktionsgeschwindigkeit zu erhalten, muss Wasser nachdosiert werden.

Dies könnte bei Verwendung nur eines Reaktors dadurch geschehen, dass beispielsweise in einem Rohrreaktor an verschiedenen Stellen Wasser eingespeist wird. In der Praxis ist es jedoch schwierig, genau die erforderliche Wassermenge einzubringen und zu erreichen, dass sofort eine homogene Lösung entsteht. Es ist technisch einfacher und daher vorteilhaft, mehrere Reaktoren in Reihe zu schalten und die notwendige Wassermenge zwischen den Reaktoren einzuspeisen.

Da durch die Umsetzung die Konzentration an tert.-Butanol steigt, erhöht sich die Wasserlöslichkeit im Reaktoraustrag. Es kann daher nach jedem Reaktor mehr Wasser zugegeben werden, als Wasser im vorigen Reaktor umgesetzt worden ist. Erfindungsgemäß wird das verbrauchte Wasser ersetzt und darüber hinaus weniger Wasser zugesetzt als zum Erreichen der Löslichkeitsgrenze notwendig wäre. Optional kann beim letzten Reaktor davon abgewichen und dieser mit einer mit Wasser gesättigten Lösung oder mit einer Mischung, die etwas mehr Wasser enthält, als der Löslichkeit entspricht, beschickt werden. Dies hat den Vorteil, dass bei geringen Isobutenkonzentrationen der Isobuten-Umsatz bei ausreichend großen Reaktorvolumen geringfügig erhöht wird, jedoch den Nachteil, dass das tert.-Butanol/Wasser-Verhältnis für die destillative Aufarbeitung ungünstiger wird, besonders dann, wenn ein hoher Anteil an wasserfreiem tert.-Butanol gewonnen werden soll.

Die Wassermenge im Eduktgemisch stammt aus einer tert.-Butanol-Wasser-Lösung, die, abgesehen von der Anfahrphase, im Prozess selbst nach Abtrennen der Kohlenwasserstoffe anfällt, d. h. durch Rezyclieren eines Teil des Reaktoraustrags. Wenn diese Wassermenge nicht ausreicht, wird zusätzlich Wasser eingespeist. Zwischen den Reaktoren kann reines Wasser oder auch seine Mischung mit tert.-Butanol eingebracht werden. Es kann auch ein Teil des durch die Umsetzung erhaltenen TBA zur Herstellung eines homogenen Gemisches mit Wasser und dem Isobutenhaltigen Kohlenwasserstoffgemisch rezycliert werden. Zur Veränderung des Gleichgewichts der Reaktion kann zwischen den Reaktoren Wasser eingespeist werden.

Erfindungsgemäß werden nur homogene Lösungen den Reaktoren zugeführt werden. Daher müssen Wasser bzw. Wasser-tert.-Butanol-Lösungen mit dem Einsatz-Kohlenwasserstoffgemisch oder einem Reaktoraustrag gemischt werden, so dass bis zum Eintritt in den ersten Reaktor bzw. einer der folgenden Reaktoren eine homogene Lösung entstanden ist. Dies kann beispielsweise unter Verwendung eines Statikmischers erreicht werden. Die Einstellung der gewünschten Wasserkonzentration im Reaktorzulauf erfolgt durch Mengenregelung der einzelnen Ströme nach Messung deren Wassergehalte.

Das Verfahren gemäß der Erfindung kann in chargenweise oder kontinuierlich arbeitenden Reaktoren, die üblicherweise bei Feststoff/Flüssigkeits-Kontaktreaktionen zum Einsatz gelangen, durchgeführt werden. Bei der Verwendung von kontinuierlich arbeitenden Strömungsreaktoren bedient man sich meistens, jedoch nicht ausschließlich, eines Festbetts. Wenn ein Festbett-Strömungsreaktor verwendet wird, kann die Flüssigkeit aufwärts oder abwärts strömen. Meistens wird ein Abwärtsströmen der Flüssigkeit bevorzugt.

Weiterhin ist es möglich, den Reaktor unter Produktrückführung oder im geraden Durchgang zu betreiben.

Bei der Verwendung von Rohrreaktoren kann das Verhältnis von Länge zu Durchmesser der Katalysatorschüttung variiert werden, entweder durch die geometrischen Maße des Reaktors oder durch dessen Füllgrad. Bei gleicher Kontaktmenge und Belastung (LHSV) können somit unterschiedliche Leerrohrgeschwindigkeiten erreicht werden. Reaktoren, bei denen ein Teil des Reaktionsgemisches zurückgeführt wird, können mit Leerrohrgeschwindigkeiten von typischerweise 13 bis 26 m/h betrieben werden. In den Reaktoren, die im geraden Durchgang durchströmt werden, können die Leerrohrgeschwindigkeiten typischerweise im Bereich von 1 bis 13 m/h liegen.

Dementsprechend beträgt die Katalysatorbelastung (LHSV) bei Reaktoren, die unter Produktrückführung betrieben werden, 0,3 bis 10 h$^{-1}$, insbesondere 1 bis 5 h$^{-1}$. Bei Reaktoren, die im geraden Durchgang durchströmt werden, liegen die Belastungen im Bereich von 0,1 bis 5,0 h$^{-1}$, insbesondere im Bereich von 0,4 bis 3 h$^{-1}$.

Das erfindungsgemäße Verfahren kann in einem, aber auch in mehreren, insbesondere 2, 3 oder 4 hintereinandergeschalteten Reaktoren, die in Fließrichtung sinkende Temperaturen aufweisen können, durchgeführt werden.

Wird der erste Reaktor oder mehrere Reaktoren unter Produktrückführung betrieben, wird ein Kreislauffaktor (Verhältnis vom im Kreis gepumpten Menge zu Frischzulauf) von 0,1 bis 10 eingestellt. Dabei beträgt der Kreislauffaktor für den ersten Reaktor bevorzugt 1 bis 4, insbesondere 2 bis 3,5.

Eine bevorzugte Verfahrensvariante ist, den ersten Reaktor unter Produktrückführung und die weiteren Reaktoren im geraden Durchgang zu betreiben. Die Anzahl der verwendeten Reaktoren liegt abhängig vom angestrebten Umsatz typischerweise zwischen 2 und 10, insbesondere zwischen 2 und 4.

Jeder Reaktor kann adiabatisch oder praktisch isotherm, d. h., mit einem Temperaturanstieg unter 10 °C betrieben werden. Ein zu hoher Temperaturanstieg ist wegen der ungünstigen Gleichgewichtsbeeinflussung (Rückspaltung) zu vermeiden.

Die Temperaturen, bei denen das erfindungsgemäße Verfahren durchgeführt wird, liegen zwischen 30 und 120 °C. Bei tieferen Temperaturen ist die Reaktionsgeschwindigkeit zu gering und bei höheren treten vermehrt Nebenreaktionen, wie beispielsweise die Oligomerisierung der Olefine auf. Vorzugsweise werden die Reaktoren im Temperaturbereich 35 bis 70 °C betrieben. Die Temperaturen in verschiedenen Reaktoren können gleich oder verschieden innerhalb des angegebenen Bereichs sein. Eine Verfahrensvariante besteht darin, die Temperatur in Flussrichtung von Reaktor zu Reaktor zu senken. Da mit sinkender Temperatur die Gleichgewichtslage günstiger wird, kann somit ein höherer Umsatz erreicht werden. Allerdings ist es nicht sinnvoll, die Temperatur unter 35 °C zu senken, da dann die Reaktion für ein technisches Verfahren zu langsam wird.

Beispielsweise kann bei vier hintereinander geschalteten Reaktoren der erste bei einer mittleren Temperatur von 67 bis 70 °C, der zweite bei einer von 53 bis 56 °C, der dritte bei einer von 42 bis 46 °C und der vierte Reaktor bei 42 bis 38 °C betrieben werden.

Die erfindungsgemäße Umsetzung kann bei einem Druck gleich oder über dem Dampfdruck des Einsatz-Kohlenwasserstoffgemisches bei der jeweiligen Reaktionstemperatur durchgeführt werden, vorzugsweise bei einem Druck unter 40 bar. Um in den Reaktoren Verdampfungsprobleme zu vermeiden, sollte der Druck 2 bis 4 bar höher als der Dampfdruck des Reaktionsgemisches sein.

Der Gesamtumsatz an Isobuten hängt von der Art und Menge des verwendeten Katalysators, den eingestellten Reaktionsbedingungen und Anzahl der Reaktionsstufen ab. Aus wirtschaftlichen Gründen wird der Isobuten-Umsatz im Bereich von 50 bis 95 %, vorzugsweise zwischen 70 und 90 % gehalten. Darüber hinaus ist es sinnvoll, um eine hohe Raum-Zeit-Ausbeute zu erhalten, Kohlenwasserstoffe mit einem Isobutengehalt nicht unter 20 %, vorzugsweise nicht unter 40 % einzusetzen.

Das den letzten Reaktor verlassende Reaktionsgemisch wird einer Destillationskolonne zugeführt, die bei oder unter dem Druck des letzten Reaktors, jedoch mindestens bei oder einem Druck von über 1 bar arbeitet. Bei der Destillation fällt als Kopfprodukt ein Kohlenwasserstoffgemisch an, das aus nicht umgesetztem Isobuten und aus den mit dem Edukt eingebrachten "inerten" Kohlenwasserstoffen besteht. Als Sumpfprodukt wird eine wässrige tert.-Butanol-Lösung gewonnen.

Das abgetrennte Kohlenwasserstoffgemisch kann zu weiteren Wertprodukten aufgearbeitet werden. Wurde beispielsweise Raffinat I oder selektivhydrierter C$_4$-Schnitt als Edukt eingesetzt, so enthält das Kopfprodukt neben nicht umgesetztem Isobuten, lineare Butene sowie Isobutan und n-Butan. Aus diesem Gemisch kann das restliche Isobuten durch Umsetzung mit Methanol zu Methyl-tert.-butylether abgetrennt werden. Im verbleibenden Raffinat können die linearen Butene - optional nach Abtrennung von 1-Buten - zu Di-n-buten und ihren höheren Oligomeren umgesetzt werden. Eine andere Verwendung des isobuten-freien Gemisches ist die Aufarbeitung zu reinem 1-Buten.

Ein Teil der gewonnenen wässrigen tert.-Butanollösung kann in den Prozess zurückgefahren (rezycliert) werden. Der andere Teil kann als solcher verwendet werden oder auf reines tert.-Butanol und einem Azeotrop aus Wasser und tert.-Butanol aufgearbeitet werden. Weiterhin ist es möglich einen anderen Strom, bestehend aus Wasser und tert.-Butanol, der bei der Aufarbeitung des Roh-tert.-Butanol anfällt, in den ersten Reaktor zurückzuführen.

Der Kreislauffaktor des auf diese Weise rezyclierten tert.-Butanols liegt bevorzugt zwischen 0,1 und 1,7.

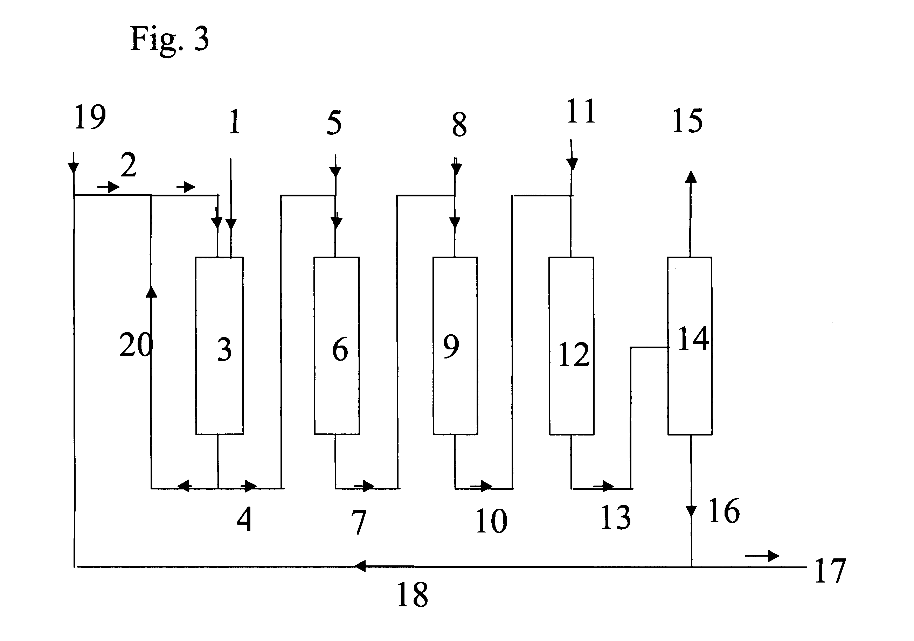

Ein Blockschema einer Anlage mit vier Reaktoren, in der das erfindungsgemäße Verfahren kontinuierlich durchgeführt werden kann, zeigt Figur 3. In den ersten Reaktor 3 werden ein Isobuten-haltiger Kohlenwasserstoffgemisch 1 (zum Beispiel Raffinat I), Austrag 20 aus dem ersten Reaktor 3 und ein tert.-Butanol/Wasser-Gemisch 2, das aus einem Rückführstrom 18 und gegebenenfalls Wasser 19 besteht, eingespeist. Der Reaktoraustrag 4 wird zum Teil wieder in den ersten Reaktor 3 zurückgeführt, der andere Teil wird zusammen mit Wasser 5 in den zweiten Reaktor 6 geleitet. Der Reaktoraustrag 7 wird zusammen mit Wasser 8 in den Reaktor 9 geführt. Die Reaktionslösung 10 wird zusammen mit Wasser 11 im Reaktor 12 umgesetzt. Der Reaktoraustrag wird in der Kolonne 14 fraktioniert. Als Kopfprodukt 15 fällt ein Kohlenwasserstoffgemisch an, das aus den unter Reaktionsbedingungen inerten Kohlenwasserstoffen und aus geringen Mengen an nicht umgesetztem Isobuten besteht. Das Sumpfprodukt 16 enthält im Wesentlichen tert.-Butanol und Wasser. Ein Teil (18) davon wird in den ersten Reaktor 3 zurückgeführt, der andere Teil (17) wird als solcher verwendet oder in einer nicht dargestellten Destillation zu tert.-Butanol/Wasser-Azeotrop und/oder tert.-Butanol aufgearbeitet.

Die folgenden Beispiele sollen die Erfindung weiter erläutern, nicht aber ihren Anwendungsbereich beschränken, der sich aus der Beschreibung und den Patentansprüchen ergibt.

Beispiele:

Der für die Versuche 1 bis 6 eingesetzte Raffinatstrom hatte die folgende Zusammensetzung:

| | |
|---|---|
| n-Butan: | 8,2 % |
| Iso-Butan: | 2,3 % |
| 1-Buten: | 30,3 % |
| 2-Buten (cis + trans): | 14,2 % |
| Isobuten: | 45,0 % |

Der Isobuten-Gehalt im Raffinat liegt typischerweise im Bereich von 30 bis 60 %.

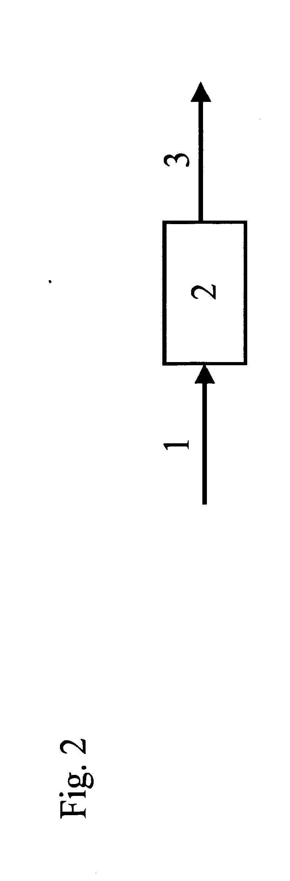

Dieser Raffinatstrom wurde in der bestehenden Produktionsanlage mit Wasser und Rück-TBA als Lösungsvermittler umgesetzt. Der Zulauf der Reaktoren der Produktionsanlage wurde als Zulauf für die Laborversuche benutzt. Figur 2 zeigt schematisch die realisierten Laboranlage.

Der Zulauf (1) wurde auf die gewünschte Temperatur vorgeheizt und in den isotherm bei 60 °C, bei den Versuchen 3 und 4 davon abweichend 55 °C, betriebenen Laborreaktor (2) geleitet. Der Reaktor bestand aus einem Katalysatorfestbett mit einem Durchmesser von 1 cm und einer Länge von 20 cm, bei den Versuchen 3 und 4 davon abweichend mit einer Länge von 25 cm. Als Katalysator wurde Amberlyst 35 in $H^+$-Form eingesetzt. Die Katalysatormenge wurde bewußt gering gehalten, um den Effekt des Wassergehalts genauer zu untersuchen. Der Produktstrom (3) wurde gaschromatographisch analysiert. Die Komponenten n-Butan, Iso-Butan, 1-Buten, 2-Buten (cis + trans) werden in Summe als Rest-$C_4$ aufgelistet. Die angegebenen Zusammensetzungen bezogen sich auf Produktausträge im quasistationären Gleichgewicht, das _ nach einer Versuchsdauer von 20 bis 30 Stunden erreicht worden war.

Der Druck der Anlage betrug 12 bar, absolut.

## 1. Beispiel (gemäß der Erfindung)

Die Stromnummern in der folgenden Tabelle waren die gleichen wie in Figur 2. Prozentangaben sind als Massen-% zu lesen. Der Zulauf (1) entsprach bezüglich des Isobutengehalts dem Zulauf zum ersten Reaktor der Produktionsanlage. Die Zulaufmenge betrug 0.3 kg/h.

| Stromnummer | Strombezeichnung | Massen-Pozent |
|---|---|---|
| 1 | Reaktoreingang | 21,87 % Isobuten<br>1,00% Wasser<br>33,40 % TBA<br>43,49 % Rest-$C_4$<br>0,23 % Sonstige |
| 3 | Reaktorausgang | 20,72 % Isobuten<br>0,63 % Wasser<br>34,92 % TBA<br>43,49 % Rest-$C_4$<br>0,24 % Sonstige |

Der erzielte Umsatz betrug: 5,30 %

Der Anteil an Wasser im Reaktorzulauf im Vergleich zum Massenanteil Wasser an der Mischungslücke betrug 35,39 %.

**Beispiel 2 (Vergleich)**

Die Stromnummern in der folgenden Tabelle waren die gleichen wie in Figur 2. Prozentangaben sind als Massen-% zu lesen. Der Zulauf (1) entsprach dem Zulauf zum ersten Reaktor der Produktionsanlage. Die Zulaufmenge betrug 0,3 kg/h.

| Stromnummer | Strombezeichnung | Massen-Pozent |
|---|---|---|
| 1 | Reaktoreingang | 21,58 % Isobuten<br>2,31 % Wasser<br>32,96 % TBA<br>42,92 % Rest-$C_4$<br>0,23 % Sonstige |
| 3 | Reaktorausgang | 20,61 % Isobuten<br>2,00 % Wasser<br>34,25 % TBA<br>42,92 % Rest-$C_4$<br>0,23 % Sonstige |

Der erzielte Umsatz betrug: 4,53 %
Der Anteil an Wasser im Reaktorzulauf im Vergleich zum Massenanteil Wasser an der Mischungslücke betrug 83,66 %.

**Beispiel 3 (gemäß der Erfindung)**

Die Stromnummern in der folgenden Tabelle waren die gleichen wie in Figur 2. Prozentangaben sind als Massen-% zu lesen. Der Zulauf (1) entsprach bezüglich des Isobutengehalts dem Zulauf zum zweiten Reaktor der Produktionsanlage. Die Zulaufmenge betrug 0.3 kg/h. Im Unterschied zu den übrigen Beispielen betrug die Reaktionstemperatur (analog der Produktionsanlage) 55 °C. Ebenfalls im Unterschied zu den übrigen Beispielen umfasste das Katalysatorbett eine Länge von 25 cm bei einem Durchmesser von 1 cm.

| Stromnummer | Strombezeichnung | Massen-Pozent |
|---|---|---|
| 1 | Reaktoreingang | 15,42 % Isobuten<br>2,00 % Wasser<br>40,21 % TBA<br>42,06 % Rest-$C_4$<br>0,31 % Sonstige |
| 3 | Reaktorausgang | 14,54 % Isobuten<br>1,72% Wasser<br>41,38 % TBA<br>42,05 % Rest-$C_4$<br>0,31 % Sonstige |

Der erzielte Umsatz betrug: 5,73 %
Der Anteil an Wasser im Reaktorzulauf im Vergleich zum Massenanteil Wasser an der Mischungslücke betrug 49,38 %.

**Beispiel 4 (Vergleich)**

Die Stromnummern in der folgenden Tabelle waren die gleichen wie in Figur 2. Prozentangaben sind als Massen-% zu lesen. Der Zulauf (1) entsprach dem Zulauf zum zweiten Reaktor der Produktionsanlage. Die Zulaufmenge betrug 0,3 kg/h. Im Unterschied zu den übrigen Beispielen betrug die Reaktionstemperatur (analog der Produktionsanlage) 55 °C. Ebenfalls im Unterschied zu den übrigen Beispielen umfasste das Katalysatorbett eine Länge von 25 cm bei einem Durchmesser von 1 cm.

| Stromnummer | Strombezeichnung | Massen-Pozent |
|---|---|---|
| 1 | Reaktoreingang | 15,21 % Isobuten<br>3,31 % Wasser<br>39,68 % TBA<br>41,49 % Rest-$C_4$<br>0.30 % Sonstige |
| 3 | Reaktorausgang | 14,38 % Isobuten<br>3,04 % Wasser<br>40,77 % TBA<br>41,49 % Rest-$C_4$<br>0,31 % Sonstige |

Der erzielte Umsatz betrug: 5.47 %
Der Anteil an Wasser im Reaktorzulauf im Vergleich zum Gewichtsanteil Wasser an der Mischungslücke betrug 84,07 %.

**Beispiel 5 (gemäß der Erfindung)**

Die Stromnummern in der folgenden Tabelle waren die gleichen wie in Figur 2. Prozentangaben sind als Massen-% zu lesen. Der Zulauf (1) entsprach bezüglich des Isobutengehalts dem Zulauf zum ersten Reaktor der Produktionsanlage, allerdings bei einem Isobuten-Massenanteil von 50 % im Raffinat I. Die Zulaufmenge betrug 0,3 kg/h.

| Stromnummer | Strombezeichnung | Massen-Pozent |
|---|---|---|
| 1 | Reaktoreingang | 25,38 % Isobuten<br>1,00 % Wasser<br>33,38 % TBA<br>39,26 % Rest-$C_4$<br>0,97 % Sonstige |
| 3 | Reaktorausgang | 23,90 % Isobuten<br>0,53 % Wasser<br>35,32 % TBA<br>39,26 % Rest-$C_4$<br>0,98 % Sonstige |

Der erzielte Umsatz betrug: 5,82 %
Der Anteil an Wasser im Reaktorzulauf im Vergleich zum Gewichtsanteil Wasser an der Mischungslücke betrug 35,56 %.

**Beispiel 6 (Vergleich)**

Die Stromnummern in der folgenden Tabelle waren die gleichen wie in Figur 2. Prozentangaben sind als Massen-% zu lesen. Der Zulauf (1) entsprach dem Zulauf zum ersten Reaktor der Produktionsanlage, allerdings bei einem Isobuten-Massenanteil von 50 % im Raffinat I. Die Zulaufmenge betrug 0,3 kg/h.

| Stromnummer | Strombezeichnung | Massen-Pozent |
|---|---|---|
| 1 | Reaktoreingang | 25,00 % Isobuten<br>2,50 % Wasser<br>32,88 % TBA<br>38,67 % Rest-$C_4$<br>0,96 % Sonstige |

(fortgesetzt)

| Stromnummer | Strombezeichnung | Massen-Pozent |
|---|---|---|
| 3 | Reaktorausgang | 23,83 % Isobuten<br>2,13 % Wasser<br>34,42 % TBA<br>38,67 % Rest-$C_4$<br>0,96 % Sonstige |

Der erzielte Umsatz betrug: 4,67 %

Der Anteil an Wasser im Reaktorzulauf im Vergleich zum Massenanteil Wasser an der Mischungslücke betrug 91,09 %.

**Beispiel 7 (gemäß der Erfindung)**

Die Stromnummern in der folgenden Tabelle waren die gleichen wie in Figur 2. Prozentangaben sind als Massen-% zu lesen. Der Zulauf (1) entsprach bezüglich des Isobutengehalts dem Zulauf zum ersten Reaktor der Produktionsanlage, allerdings bei einem Isobuten-Massenanteil von 60 % im Raffinat I. Die Zulaufmenge betrug 0,3 kg/h.

| Stromnummer | Strombezeichnung | Massen-Pozent |
|---|---|---|
| 1 | Reaktoreingang | 32,67 % Isobuten<br>1,21 % Wasser<br>33,32 % TBA<br>31,50 % Rest-$C_4$<br>1,30 % Sonstige |
| 3 | Reaktorausgang | 30,52 % Isobuten<br>0,52 % Wasser<br>36,14 % TBA<br>31,50 % Rest-$C_4$<br>1,32 % Sonstige |

Der erzielte Umsatz betrug: 6,58 %

Der Anteil an Wasser im Reaktorzulauf im Vergleich zum Gewichtsanteil Wasser an der Mischungslücke betrug 42,88 %.

**Beispiel 8 (gemäß der Erfindung)**

Die Stromnummern in der folgenden Tabelle waren die gleichen wie in Figur 2. Prozentangaben sind als Massen-% zu lesen. Der Zulauf (1) entsprach dem Zulauf zum ersten Reaktor der Produktionsanlage, allerdings bei einem Isobuten-Massenanteil von 60 % im Raffinat I. Die Zulaufmenge betrug 0,3 kg/h.

| Stromnummer | Strombezeichnung | Massen-Pozent |
|---|---|---|
| 1 | Reaktoreingang | 32,34 % Isobuten<br>2,20 % Wasser<br>32,99 % TBA<br>31,18 % Rest-$C_4$<br>1,29% Sonstige |
| 3 | Reaktorausgang | 30,63 % Isobuten<br>1,66 % Wasser<br>35,24 % TBA<br>31,18 % Rest-$C_4$<br>1,29 % Sonstige |

Der erzielte Umsatz betrug: 5,28 %

Der Anteil an Wasser im Reaktorzulauf im Vergleich zum Massenanteil Wasser an der Mischungslücke betrug 79,72 %.

**Beispiel 9 (Vergleich)**

Die Stromnummern in der folgenden Tabelle waren die gleichen wie in Figur 2. Prozentangaben sind als Massen-% zu lesen. Der Zulauf (1) entsprach dem Zulauf zum ersten Reaktor der Produktionsanlage, allerdings bei einem Isobuten-Massenanteil von 60 % im Raffinat I. Die Zulaufmenge betrug 0,3 kg/h.

| Stromnummer | Strombezeichnung | Massen-Pozent |
|---|---|---|
| 1 | Reaktoreingang | 32,17 % Isobuten<br>2,71 % Wasser<br>32,82 % TBA<br>31,02 % Rest-$C_4$<br>1,28 % Sonstige |
| 3 | Reaktorausgang | 30,61 % Isobuten<br>2,21 % Wasser<br>34,88 % TBA<br>31,02 % Rest-$C_4$<br>1,29 % Sonstige |

Der erzielte Umsatz betrug: 4,87 %

Der Anteil an Wasser im Reaktorzulauf im Vergleich zum Massenanteil Wasser an der Mischungslücke betrug 98,83 %.

**Beispiel 10 (Vergleich)**

Die Stromnummern in der folgenden Tabelle waren die gleichen wie in Figur 2. Prozentangaben sind als Massen-% zu lesen. Der Zulauf (1) entsprach dem Zulauf zum ersten Reaktor der Produktionsanlage, allerdings bei einem Isobuten-Massenanteil von 60 % im Raffinat I. Die Zulaufmenge betrug 0,3 kg/h.

| Stromnummer | Strombezeichnung | Massen-Pozent |
|---|---|---|
| 1 | Reaktoreingang | 32,84 % Isobuten<br>0,70 % Wasser<br>33,49 % TBA<br>31,66 % Rest-C4<br>1,31 % sonstige |
| 3 | Reaktorausgang | 31,17 % Isobuten<br>0,20 % Wasser<br>35,57 % TBA<br>31,66 % Rest-$C_4$<br>1,40 % sonstige |

Der erzielte Umsatz betrug: 5,06 %

Der Anteil an Wasser im Reaktorzulauf im Vergleich zum Massenanteil Wasser an der Mischungslücke betrug 24,79 %.

Bei Vergleich von Versuch 1 mit Versuch 2, von Versuch 3 mit Versuch 4, von Versuch 5 mit Versuch 6 und bei Vergleich der Versuche 7, 8 und 9 ergibt sich, dass jeweils die Versuche mit dem geringsten Wassergehalt die höchsten Isobutenumsätze zu TBA ergeben.

Der Vergleich der Beispiele 7, 8 und 10 zeigen, dass beim Unterschreiten der beanspruchten Grenze von mindestens 30 % Wasser bezogen auf den Massenanteil Wasser an der Mischungslücke der erzielbare Umsatz wieder fällt.

## EP 1 431 264 B1

**Patentansprüche**

1. Verfahren zur Herstellung von tert.-Butanol durch Umsetzung eines homogenen Reaktionsgemisches aus Wasser, tert.-Butanol und einem Isobuten-haltigen Kohlenwasserstoffgemisch, an einem sauren Ionenaustauscherharz bei 30 bis 120 °C,
**dadurch gekennzeichnet,**
**dass** das homogene Reaktionsgemisch bei Beginn der Umsetzung einen Massenanteil von Isobuten von über 10 Massen-% und einen Wasseranteil von 30 bis 80 % der durch die Löslichkeit von Wasser im Reaktionsgemisch möglichen Wassermenge enthält.

2. Verfahren nach Anspruch 1,
**dadurch gekennzeichnet,**
**dass** ein Teil des durch die Umsetzung erhaltenen tert.-Butanols zur Herstellung des homogenen Reaktionsgemisches rezycliert wird.

3. Verfahren nach Anspruch 2,
**dadurch gekennzeichnet,**
**dass** der Kreislauffaktor zur Rezyclierung des durch die Umsetzung erhaltenen tert.-Butanols zwischen 0.1 und 1.7 beträgt.

4. Verfahren nach Anspruch 1 bis 3,
**dadurch gekennzeichnet,**
**dass** die Umsetzung in mehreren, hintereinander geschalteten Reaktoren durchgeführt wird.

5. Verfahren nach Anspruch 4,
**dadurch gekennzeichnet,**
**dass** zwischen den Reaktoren Wasser zugeführt wird.

6. Verfahren nach einem der Ansprüche 1 bis 5,
**dadurch gekennzeichnet,**
**dass** der Wasseranteil des homogenen Reaktionsgemisches 50 bis 80 % der Maximalmenge beträgt.

7. Verfahren nach einem der Ansprüche 1 bis 6,
**dadurch gekennzeichnet,**
**dass** die hintereinander geschalteten Reaktoren in Fließrichtung sinkende Temperaturen aufweisen.

**Claims**

1. Process for preparing tert-butanol by reaction of a homogeneous reaction mixture of water, tert-butanol and an isobutene-containing hydrocarbon mixture over an acidic ion-exchange resin at from 30 bis 120°C, **characterized in that** the homogeneous reaction mixture at the start of the reaction has a proportion by mass of isobutene of over 10% by mass and a proportion of water which is from 30 to 80% of the amount of water which is possible on the basis of the solubility of water in the reaction mixture.

2. Process according to Claim 1, **characterized in that** part of the tert-butanol obtained by means of the reaction is recycled to prepare the homogeneous reaction mixture.

3. Process according to Claim 2, **characterized in that** the recirculation factor for recycling of the tert-butanol obtained by means of the reaction is from 0.1 to 1.7.

4. Process according to any of Claims 1 to 3,
**characterized in that** the reaction is carried out in a plurality of reactors connected in series.

5. Process according to Claim 4, **characterized in that** water is introduced between the reactors.

6. Process according to any of Claims 1 to 5,
**characterized in that** the water content of the homogeneous reaction mixture is from 50 to 80% of the maximum amount.

**7.** Process according to any of Claims 1 to 6,
**characterized in that** the reactors connected in series have decreasing temperatures in the flow direction.

**Revendications**

**1.** Procédé pour la préparation de tert-butanol par transformation d'un mélange réactionnel homogène d'eau, de tert-butanol et d'un mélange d'hydrocarbures contenant de l'isobutène, sur une résine échangeuse d'ions acide à 30 jusqu'à 120°C, **caractérisé en ce que** le mélange réactionnel homogène contient, au début de la transformation, une proportion massique d'isobutène supérieure à 10% en masse et une proportion d'eau de 30 à 80% de la quantité d'eau possible dans le mélange réactionnel par la solubilité de l'eau.

**2.** Procédé selon la revendication 1, **caractérisé en ce qu'**une partie du tert-butanol obtenu par la transformation est recyclée pour la préparation du mélange réactionnel homogène.

**3.** Procédé selon la revendication 2, **caractérisé en ce que** le facteur de circulation pour le recyclage du tert-butanol obtenu par la transformation est compris entre 0,1 et 1,7.

**4.** Procédé selon la revendication 1 à 3, **caractérisé en ce que** la transformation est réalisée dans plusieurs réacteurs disposés l'un derrière l'autre.

**5.** Procédé selon la revendication 4, **caractérisé en ce que** de l'eau est alimentée entre les réacteurs.

**6.** Procédé selon l'une quelconque des revendications 1 à 5, **caractérisé en ce que** la proportion d'eau du mélange réactionnel homogène est de 50 à 80% de la quantité maximale.

**7.** Procédé selon l'une quelconque des revendications 1 à 6, **caractérisé en ce que** les réacteurs disposés les uns derrière les autres présentent des températures qui s'abaissent dans le sens de l'écoulement.

Fig. 1

Fig. 2

Fig. 3

EP 1 431 264 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0579153 A
- DE 3031702
- EP 0010993 A
- US 4327231 A
- WO 9933775 A
- DE 3025262
- DE 03025262

**In der Beschreibung aufgeführte Nicht-Patentliteratur**

- **E. Velo ; L. Puigjaner ; F. Recasens.** Inhibition by Product in the Liquid-Phase-Hydration of Isobutene to tert-Butyl Alcohol : Kinetic and Equilibrium Studies. *Ind. Eng. Chem.Res.,* 1988, vol. 27, 2224-2231